Europäisches Patentamt

European Patent Office

Office européen des brevets

(11) Numéro de publication: **0 336 861**
A1

## (12) DEMANDE DE BREVET EUROPEEN

(21) Numéro de dépôt: 89420119.3

(22) Date de dépôt: 31.03.89

(51) Int. Cl.⁴: **A 61 F 2/38**

(30) Priorité: 01.04.88 FR 8804783

(43) Date de publication de la demande:
11.10.89 Bulletin 89/41

(84) Etats contractants désignés:
AT BE CH DE ES GB GR IT LI LU NL SE

(71) Demandeur: **Broc, Christian**
**Les Beaumettes**
**F-84220 Gordes (FR)**

(72) Inventeur: **Broc, Christian**
**Les Beaumettes**
**F-84220 Gordes (FR)**

(74) Mandataire: **Dupuis, François**
**Cabinet Charras 3 Place de l'Hôtel-de-Ville BP 203**
**F-42005 St. Etienne Cédex 1 (FR)**

(54) **Implant fémoro-rotulien.**

(57) L'implant est remarquable en ce que l'élément trochléen (1) a une section transversale concave d'épaisseur décroissante à partir de son bord externe (1a), situé du côté du condyle externe, pour venir mourir, d'une manière tangente, au niveau de la gorge trochléenne, après fixation dudit élément pour créer un relèvement de la surface externe trochléenne.

FIG.2

EP 0 336 861 A1

**Description**

**Implant fémoro-rotulien.**

L'invention concerne les prothèses trochléo-patellaire du genou.

Le problème général posé est de tenir compte de toutes les contraintes dans le fonctionnement du genou notamment des composantes rotatoires dans le cas des prothèses du type de celles définies par l'objet de l'invention.

On connait, par le brevet français 8517203, un élément rotulien réalisé à partir d'un bouclier condylien prolongé dans un plan perpendiculaire par une partie commune, dont les extrémités latérales sont coupées en biseau, pour correspondre respectivement à la course rotulienne externe et interne.

On sait qu'au moment de la flexion du genou, il apparaît une rotation externe du fémur par rapport au tibia qui demeure fixe de sorte que la rotule en fonction de la vitesse de flexion, bascule vers l'extérieur avec une pression plus ou moins importante. Il en résulte que la face mécanique fonctionnelle de la rotule est la partie située à l'extérieur tandis que la partie située vers le centre demeure figée.

C'est donc la partie supérieure de la trochlée qui est importante car après engagement de la rotule dans la partie médiane de l'échancrure intercondylienne, elle n'a plus de mobilité et constitue simplement un point d'appel. La rotule doit donc être non contrainte et non symétrique pour éviter tout problème de luxation.

Pour résoudre ce premier problème, il a fallu donner un certain profil en section à l'élément trochléen considéré dans un plan frontal. Dans ce but, selon l'invention, l'élément trochléen a une section transversale concave d'épaisseur décroissante à partir de son bord externe situé du côté du condyle externe pour venir mourir, d'une manière tangente, au niveau de la gorge trochléenne après fixation dudit élément.

Il en résulte un relèvement de la surface mécanique externe trochléenne qui est artificiellement accentuée par la courbure de l'implant à concavité supéro-interne sur la trochlée externe, ce qui permet à la partie rotulienne de se plaquer quelle que soit sa course sur la face mécanique fémorale.

Sur un plan sagittal, l'élément trochléen doit épouser la forme du condyle en présentant un arc de courbure qui lui permet de passer de la gorge trochléenne à la partie supérieure de la trochlée. Demeure cependant un problème qui est de laisser la place dans le cas d'un implant disposé du côté externe fémoro-tibial et susceptible d'entrer en conflit avec la partie trochléo-condylienne.

Cet autre problème est résolu selon l'invention en ce que le bord transversal de l'élément trochléen, situé du côté du tibia, présente une échancrure profilée en étant apte à laisser la place à un patin supposé du côté externe fémorotibial.

Dans ces conditions, la fixation de l'élément trochléen au niveau de l'échancrure, doit être parfait pour éviter tout risque de relevage et de conflit avec les ligaments.

Pour résoudre ce problème de fixation, l'élément trochléen présente, au niveau de l'échancrure frontale profilée, au moins un plot d'ancrage et au niveau de son bord transversal opposé au moins un autre plot d'ancrage pour assurer, en combinaison, la fixation dudit élément.

Un autre inconvénient existant des éléments trochléen connus, apparaît au niveau de leur profil longitudinal qui n'est pas adapté. En effet, si un tel profil reproduit bien la courbure naturelle du condyle, après fixation de l'élément trochléen, son extrémité située du côté antérieur apparaît en surépaisseur créant un effet de butée préjudiciable.

Pour supprimer un tel effet, il est nécessaire de mettre du ciment pour créer une pente douce. On conçoit que cela n'est pas rationnel. Ou bien il est nécessaire de resséquer la partie de l'os correspondante ce qui est long et très délicat car l'accès est très difficile.

Cet autre problème posé est résolu selon l'invention en ce que le profil longitudinal de l'élément trochléen reproduit très sensiblement la courbure naturelle d'un condyle en étant terminée par une contre-courbure condylo-diaphillaire qui apparaît en tangence au niveau antérieur du fémur.

Avantageusement, l'élément trochléen selon l'invention combine les caractéristiques précédentes pour résoudre globalement les différents problèmes posés. Plus particulièrement, l'implant fémoro-rotulien comprendra un élément trochléen qui a une section transversale concave d'épaisseur décroissante à partir de son bord externe situé du côté du condyle externe, pour venir mourir, d'une manière tangente, au niveau de la gorge trochléenne, après fixation des éléments ; le profil longitudinal de l'élément trochléen reproduit très sensiblement la courbure naturelle d'un condyle en étant terminé par une contre courbure condylo-diaphillaire qui apparaît en tangence au niveau antérieur du fémur.

Suivant une autre caractéristique, la section longitudinale de l'élément trochléen a une épaisseur progressivement croissante à partir de la contre-courbure jusqu'à sensiblement la partie médiane puis progressivement décroissante jusqu'à l'extrémité de la courbure antérieure.

En ce qui concerne l'autre élément de l'implant fémoro-rotulien, à savoir l'élément rotulien, il doit être parallèle au plan condylien. Dans ce but, en combinaison avec l'élément trochlée tel que défini, l'élément rotulien selon l'invention a la forme d'une calotte hémisphérique présentant une double courbure concave sur le plan supérieur et sur le plan sagittal.

Pour éviter toute perte de subtance osseuse et résoudre ce problème, l'élément rotulien présente des plots d'ancrage pour être fixé sur la face d'appui de la rotule, en combinaison avec du ciment.

L'invention est exposée ci-après plus en détail à l'aide des dessins annexés dans lesquels :

    - la figure 1 est une vue en perspective montrant la mise en place de l'élément trochléen selon l'invention sur la partie correspondante du fémur,

- la figure 2 est une vue en coupe transversale de l'implant trochléen fixé sur la partie correspondante du fémur,

- la figure 3 est une vue en coupe longitudinale de l'implant trochléen sur le fémur,

- la figure 4 est une vue de face de l'élément trochléen,

- la figure 5 est une vue en coupe de l'élément rotulien,

- la figure 6 est une vue en plan de l'élément rotulien.

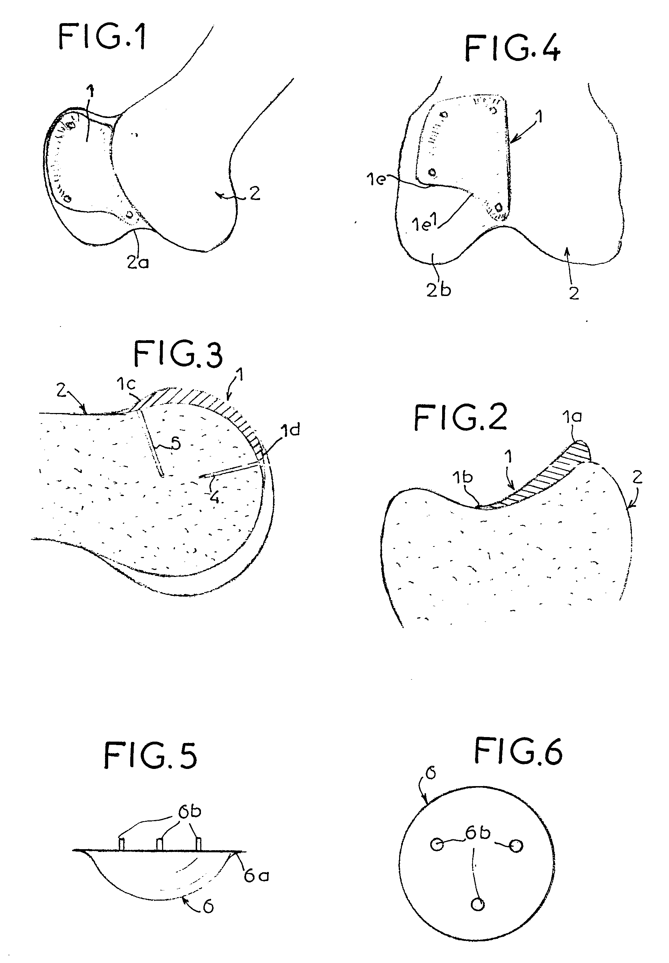

L'élément trochléen désigné dans son ensemble par (1) est destiné à être implanté, d'une manière connue, au niveau de la trochlée inter-condylienne (2a) d'un fémur (2). Selon l'invention, l'élément trochléen (1) a une section transversale concave d'épaisseur dégressive à partir de son bord externe (1a), situé du côté du condyle externe (2b) du fémur. Ce bord surélevé (1a) se raccorde avec le bord longitudinal interne (1b), d'une manière tangente, au niveau de la gorge trochléenne (2a) (figure 2). A noter que ce raccordement peut avoir lieu indifféremment avant ou après la gorge (2a). Sur un plan sagittal, l'implant devant épouser la forme du condyle, la section transversale concave décrit un arc de courbure qui lui permet de passer de l'échancrure à la partie supérieure de la trochlée. Il apparaît donc un relèvement de la surface externe trochléenne accentué par le profil de l'implant ainsi défini.

Suivant une autre caractéristique, le profil longitudinal de l'élément trochléen (1) qui reproduit la courbure naturelle du condyle, est terminé par une contre courbure condylo-diaphillaire (1c) conformé pour être située après im plantation de l'élément (1), en position de tangence avec la partie supérieure du fémur (figure 3). Cette contre-courbure (1c) qui supprime l'effet de butée, évite par conséquent d'entailler le fémur pour encastrer la partie supérieure de l'implant trochléen.

En outre, la section longitudinale de l'élément trochléen (1) a une épaisseur progressivement croissante à partir de la contre-courbure (1c) jusqu'à sensiblement la partie médiane dudit élément, puis progressivement décroissante jusqu'à l'extrémité de la courbure antérieure (1d).

Comme le montre la figure 4, le bord transversal (1e) de l'élément trochléen (1), présente une échancrure profilée (1e1) apte à laisser la place à un patin (9) supposé du côté externe fémoro-tibial, au moment de la flexion du genou qui engendre un effet rotationnel du tibia par rapport aux condyles fémoraux. Cette échancrure (1e1) évite ainsi tout risque de conflit entre le patin et la partie trochléo-condylienne.

Avantageusement, mais d'une manière non rigoureusement limitative, l'élément trochléen combine les différentes caractéristiques définies.

D'une manière importante, la fixation de l'élément trochléen, notamment au niveau de l'échancrure (1e1) doit être parfaite pour éviter tout risque de relevage et d'être en conflit avec les ligaments. A cet égard, l'élément trochléen présente au niveau de l'échancrure frontale profilée (1e1) au moins un plot d'ancrage (4) et au niveau de son bord transversal opposé au moins un autre plot d'ancrage (5) pour assurer, en combinaison, la fixation sûre et efficace dudit élément (1).

Dans certains cas, il est nécessaire de prévoir, en plus de l'élément trochléen (1) tel que défini, un élément rotulien (6). Dans ce but, l'élément rotulien (6) est réalisé à partir d'un bouton hémisphérique présentant une double courbure concave (6a) sur le plan supérieur et sur le plan sagittal. Ce bouton présente des plots d'ancrage (6b), de préférence au nombre de trois, pour être fixé sur la face d'appui de la rotule pour éviter toute perte de substance osseuse. De préférence, la fixation de l'élément rotulien (6) s'effectue en combinaison avec du ciment.

Les avantages ressortent bien de la description.

**Revendications**

- 1 - Implant fémoro-rotulien , caractérisé , en ce que l'élément trochléen (1) a une section transversale concave d'épaisseur décroissante à partir de son bord externe (1a), situé du côté du condyle externe, pour venir mourir, d'une manière tangente, au niveau de la gorge trochléenne, après fixation dudit élément pour créer un relèvement de la surface externe trochléenne.

- 2 - Implant selon la revendication 1 caractérisé en ce que la section transversale concave décrit un arc de courbure qui lui permet de passer de l'échancrure à la partie supérieure de la trochlée.

- 3 - Implant fémoro-rotulien selon la revendication 1 , caractérisé en ce que le profil longitudinal de l'élément trochléen (1) reproduit très sensiblement la courbure naturelle d'un condyle en étant terminée par une contre-courbure condylo-diaphillaire (1c) qui apparaît en tangence au niveau antérieur du fémur.

- 4 - Implant fémoro-rotulien selon l'une quelconque des revendications 1, 2 et 3, caractérisé en ce que le bord transversal (1e) de l'élément trochléen, situé du côté du tibia, présente une échancrure (1e1) profilée en étant apte à laisser la place à un patin supposé du côté externe fémoro-tibial.

-5 - Implant selon la revendication 3, caractérisé en ce que la section longitudinale de l'élément trochléen (1) a une épaisseur progressivement croissante à partir de la contre-courbure (1c) jusqu'à sensiblement la partie médiane puis progressivement décroissante jusqu'à l'extrémité de la courbure antérieure (1d).

-6 - Implant selon l'une quelconque des revendications 1 à 5, caractérisé en ce que l'élément trochléen présente, au niveau de l'échancrure frontale profilée, au moins un plot d'ancrage (4) et au niveau de son bord transversal opposé au moins un autre plot d'ancrage (5) pour assurer, en combinaison, la fixation dudit élément (1).

- 7 - Implant selon la revendication 1, caractérisé en ce que l'élément rotulien (6) a la forme d'une calotte hémisphérique présentant une double courbure concave sur le plan supérieur et sur le plan sagittal.

- 8 - Implant selon la revendication 7, caractérisé en ce que l'élément rotulien (6) présente des plots d'ancrage pour être fixé sur la face d'appui de la rotule, en combinaison avec du ciment.

FIG.1

FIG.4

FIG.3

FIG.2

FIG.5

FIG.6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | WO-A-8 702 882 (J.M. AUBANIAC) <br> * Figures 21-24 * | 1,6 | A 61 F 2/38 |
| Y | | 7,8 | |
| | --- | | |
| Y | DE-U-8 712 469 (S & G IMPLANTS GmbH) <br> * Figure 1 * | 7,8 | |
| | --- | | |
| A | FR-A-2 594 323 (MATCO S.R.L.) | 1-6 | |
| | ----- | | |

DOMAINES TECHNIQUES RECHERCHES (Int. Cl.4)

A 61 F

**Le présent rapport a été établi pour toutes les revendications**

| Lieu de la recherche | Date d'achevement de la recherche | Examinateur |
|---|---|---|
| LA HAYE | 14-07-1989 | ARGENTINI A. |

EPO FORM 1503 03.82 (P0402)